# EUROPEAN PATENT APPLICATION

(11) **EP 3 932 461 A1**
(43) Date of publication of application: **05.01.2022**
(21) Application number: 20770294.5
(22) Date of filing: 09.03.2020
(51) Int. Cl.: A61M 25/09

(54) **TORQUE DEVICE**

(30) Priority: 13.03.2019 JP 2019045466
(71) Applicant: TERUMO Kabushiki Kaisha, Tokyo 151-0072 (JP)
(72) Inventor: SHIMADA, Daisuke, Fujinomiya-shi, Shizuoka 418-0015 (JP); ITO, Takashi, Fujinomiya-shi Shizuoka 418-0051 (JP); ARAHIRA, Shouta, Fujinomiya-shi Shizuoka 418-0051 (JP)
(74) Representative: Casalonga
(86) International application number: PCT/JP2020/009969
(87) International publication number: WO 2020/184507

(57) **Abstract**

Provided is a torque device that has good manual operability and easily attaches to an elongated body such as a guide wire.

A torque device (20) for holding an elongated body (10) comprises a first member (30) and a second member (31) in which is formed a connected space portion (35) through which the elongated body (10) can be inserted in the length direction. The first member (30) and the second member (31) are able to rotate with each other in a state in which the elongated body (10) is being held, and have an opening portion (36) that allows the space portion (35) to communicate with the outside in the radial direction. The opening portion (36) is continuous with the first member (30) and the second member (31) in the longitudinal direction, and has a width that allows the elongated body (10) to be inserted therethrough. At least one of the first member (30) and the second member (31) has a diameter decreasing portion (45) that reduces the diameter of the space portion (35) as a result of the first member (30) and the second member (31) rotating with each another.

## Description

### Technical Field

The present invention relates to a torque device that improves the operability of an elongated body such as a guide wire inserted into a living body.

### Background Art

When a catheter is inserted into a biological lumen, a guide wire is used as an elongated body that guides the catheter to a target site in the biological lumen. The guide wire is inserted along an axial direction of the catheter, and is inserted into the biological lumen prior to the catheter to guide the insertion of the catheter. The guide wire is also used to guide an endoscope. Since the biological lumen is bent and branched, it is necessary to move the guide wire forward and backward and rotate the guide wire when inserting the guide wire into the biological lumen.

Since the guide wire has a small diameter as it is, it is difficult to hold and operate the guide wire. Therefore, there is known a torque device attached to a hand portion of the guide wire to facilitate operation. Examples of the torque device include those described in PTL 1.

### Citation List

### Patent Literature

PTL 1: JP-A-2011-62522

### Summary of Invention

### Technical Problem

It is desirable that the torque device is easy to attach to the guide wire and has good manual operability, and a torque device that has both of these is desired.

The invention has been made to solve the above-described problems, and an object of the invention is to provide a torque device that can be easily attached to an elongated body such as a guide wire and has good manual operability.

### Solution to Problem

A torque device that holds an elongated body according to the invention that achieves the above object includes:
a first member and a second member that define a continuous space portion through which the elongated body is inserted along a length direction, in which
the first member and the second member are rotatable relative to each other while holding the elongated body, and have an opening portion that connects the space portion and outside in a radial direction,
the opening portion is continuous in a length direction of the first member and the second member, and has a width through which the elongated body is insertable, and
at least one of the first member and the second member includes a diameter decreasing portion that decreases a diameter of the space portion by the first member and the second member rotating relative to each other.

### Advantageous Effect of Invention

The torque device configured as described above can easily guide the elongated body from the opening portion to the space portion, and can hold the elongated body disposed in the space portion by the first member and the second member rotating relative to each other, and therefore, the elongated body can be easily attached and held. Further, the elongated body attached and held by the torque device can be moved forward and backward and rotated, and the operability can be improved.

The first member and the second member may move along the length direction of the elongated body by rotating relative to each other, and the diameter decreasing portion of one of the first member and the second member may be decreased in diameter by the other one of the first member and the second member moving along the length direction of the elongated body. Accordingly, it is possible to form a structure in which the diameter decreasing portion is decreased with a simple structure of forming a screw surface on the first member and the second member.

The diameter decreasing portion may be elastically deformable in the radial direction, and may have a pressing surface portion that is pressed in the radial direction by the other one of the first member and the second member when the first member and the second member move along the length direction of the elongated body by rotating relative to each other. Accordingly, the elongated body can be elastically held by the diameter decreasing portion, and the operability of the held elongated body can be further improved.

At least one of the first member and the second member may have an elastically deformable inner peripheral member that is in pressure contact with or joined to the other one of the first member and the second member, and the inner peripheral member may have an inner peripheral surface that constitutes at least a part of a peripheral surface of the space portion to form the diameter decreasing portion. Accordingly, by the first member and the second member rotating relative to each other, it is possible to form a structure in which the inner peripheral member is twisted to decrease the diameter.

The inner peripheral member provided on one of the first member and the second member may include a protruding portion that enters the other one of the first member and the second member. Accordingly, the inner peripheral surface of the inner peripheral member can be enlarged, and the elongated body can be held more firmly.

The opening portion may be continuous in the length direction of the elongated body over the first member and the second member in a state where the diameter decreasing portion is enlarged. Accordingly, the elongated body can be easily inserted into the space portion through the opening portion.

### Brief Description of Drawings

[Fig. 1] Fig. 1 is a perspective view of a torque device according to a first embodiment.
[Fig. 2] Fig. 2 is a cross-sectional view of the torque device of Fig. 1.
[Fig. 3] Fig. 3 is a cross-sectional view of the torque device in a state where a diameter of a diameter decreasing portion is decreased.
[Fig. 4] Fig. 4 is a cross-sectional view of the torque device in a state where a guide wire is held by the diameter decreasing portion.
[Fig. 5] Fig. 5 is a cross-sectional view of a torque device according to a second embodiment.
[Fig. 6] Fig. 6 is a cross-sectional view of a torque device in a state where a diameter of a diameter decreasing portion of Fig. 5 is decreased.
[Fig. 7] Fig. 7 is an enlarged cross-sectional view of a part of a rotation latch mechanism.
[Fig. 8] Fig. 8 is a cross-sectional view of a torque device according to a third embodiment.

### Description of Embodiments

Embodiments of the invention will be described below with reference to the drawings. Note that dimensional ratios in the drawings may be exaggerated for convenience of description and may differ from the actual ratios.

A torque device 20 according to an embodiment of the invention is attached to a hand portion of a guide wire 10 to be inserted into a biological lumen to improve the operability of the guide wire 10 by an operator.

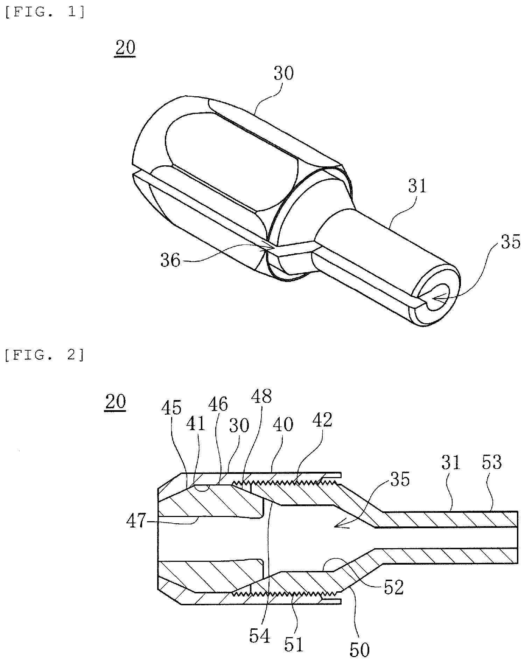

As shown in Fig. 1, the torque device 20 of the present embodiment includes a first member 30 and a second member 31 integrated with each other. Further, the torque device 20 is formed with a continuous space portion 35 through the entire length. The guide wire 10 can be inserted through the space portion 35 in a state where the first member 39 and the second member 31 are integrated. In addition, the torque device 20 has an opening portion 36 through the entire length. The opening portion 36 connects the space portion 35 and the outside in a radial direction. The opening portion 36 has a width for the guide wire 10 to be inserted into the space portion 35 from the outside.

The first member 30 and the second member 31 are rotatable relative to each other and move relative to each other along a length direction with the rotation. By moving the first member 30 and the second member 31 relative to each other in the length direction, it is possible to hold the guide wire 10 inserted through the space portion 35 to be described later. The guide wire 10 can be introduced from the opening portion 36 into the space portion 35 in a state where the first member 30 and the second member 31 are disposed such that the opening portion 36 is continuous through the entire length as shown in Fig. 1.

As shown in Fig. 2, the first member 30 has an outer peripheral surface 40 facing the outside and an inner peripheral surface 41 facing the space portion 35. The inner peripheral surface 41 is formed with an inner peripheral screw surface 42 that is a screw thread for screwing the second member 31.

The first member 30 includes a tubular diameter decreasing portion 45 on an end portion in the space portion 35. The diameter decreasing portion 45 is made of an elastic material such as rubber. An outer peripheral surface 46 of the diameter decreasing portion 45 is abutted against the inner peripheral surface 41 of the first member 30. Further, an inner peripheral surface 47 of the diameter decreasing portion 45 has a diameter larger than that of the guide wire 10, and the guide wire 10 can be inserted in a state shown in Figs. 1 and 2. Similar to the first member 30 and the second member 31, the diameter decreasing portion 45 has an opening portion through the entire length at one part in a circumferential direction (not shown), and the guide wire 10 can be inserted in the radial direction.

The second member 31 has an outer peripheral surface 50 having a diameter that substantially matches an inner diameter of the first member 30. An outer diameter of the second member 31 on an end portion side exposed from the first member 30 is smaller than that of the outer peripheral surface 50 in the first member 30. The outer peripheral surface 50 of the second member 31 has an outer peripheral screw surface 51 screwed to the inner peripheral screw surface 42 of the first member 30.

The second member 31 is formed with a tapered surface portion 54 at an end portion in the first member 30. The tapered surface portion 54 has an inner diameter increasing toward an end surface side. The tapered surface portion 54 is abutted against a pressing surface portion 48 that is an end portion outer peripheral surface of the diameter decreasing portion 45. The pressing surface portion 48 of the diameter decreasing portion 45 is inclined in the same direction as the tapered surface portion 54, and is pressed toward an inner peripheral side by the tapered surface portion 54 when the pressing surface portion 48 moves in the length direction of the guide wire 10.

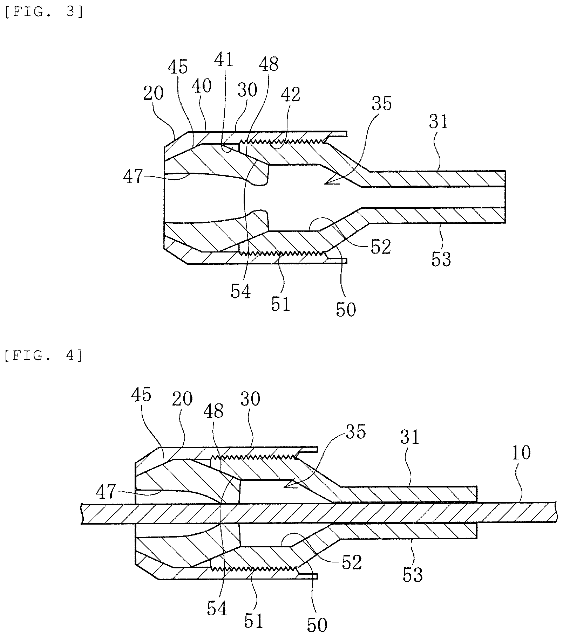

As shown in Fig. 3, when the first member 30 and the second member 31 are rotated relative to each other and the second member 31 is moved in the length direction to enter the inside of the first member 30, the pressing surface portion 48 of the diameter decreasing portion 45 is pressed toward the inner peripheral side by the tapered surface portion 54 of the second member 31. Accordingly, the diameter decreasing portion 45 is deformed toward the inner peripheral side, and the inner diameter becomes smaller.

As shown in Fig. 4, when the first member 30 and the second member 31 are rotated relative to each other in a state where the guide wire 10 is inserted, the guide wire 10 is held by the diameter decreasing portion 45. Accordingly, the guide wire 10 is fixed in the length direction and the radial direction with respect to the torque device 20, and the operator can move the guide wire 10 forward and backward and can rotate the guide wire 10 via the torque device 20.

When the torque device 20 is used, a positional relationship between the first member 30 and the second member 31 is set to the state shown in Fig. 2 in advance. In this state, the opening portion 36 is continuous over the first member 30 and the second member 31 as shown in Fig. 1, and the guide wire 10 can be inserted into the space portion 35 through the opening portion 36. After the guide wire 10 is inserted into the space portion 35, a position of the torque device 20 in the length direction of the guide wire 10 is adjusted. After the position of the torque device 20 is determined, the first member 30 and the second member 31 are rotated relative to each other into a state shown in Fig. 4. Accordingly, the guide wire 10 of the space portion 35 is held by the diameter decreasing portion 45. When the guide wire 10 is held by the diameter decreasing portion 45, the guide wire 10 can be moved forward and backward and rotated via the torque device 20.

When the torque device 20 is removed from the guide wire 10, the first member 30 and the second member 31 are rotated relative to each other to change from the state of Fig. 4 to the state of Fig. 2. Accordingly, a state of the diameter decreasing portion 45 of holding the guide wire 10 is released, and the guide wire 10 can be removed from the space portion 35 to the outside through the opening portion 36.

As described above, in the torque device 20 of the present embodiment, the guide wire 10 can be inserted in the radial direction from the opening portion 36 which is continuous over the first member 30 and the second member 31, and the inserted guide wire 10 can be held by the diameter decreasing portion 45 by rotating the first member 30 and the second member 31 relative to each other. Therefore, the guide wire 10 can be easily attached. Since the first member 30 and the second member 31 constituting the torque device 20 are rotary bodies, the torque device 20 holding the guide wire 10 can be easily rotated.

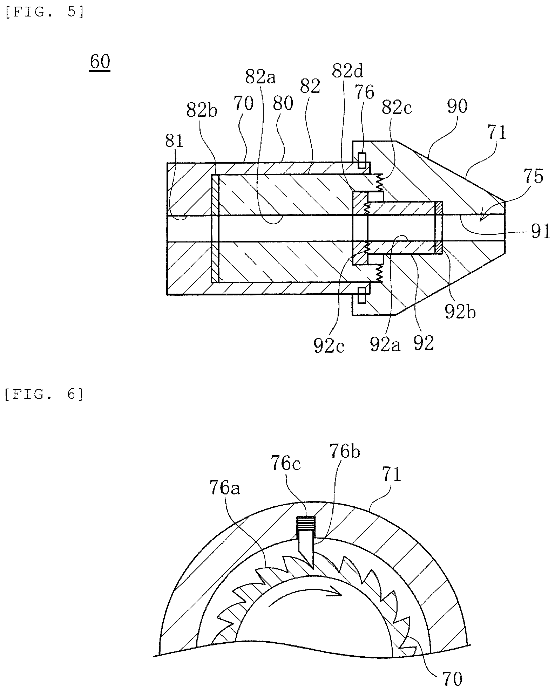

Next, a second embodiment of the invention will be described. As shown in Fig. 5, a torque device 60 of the second embodiment includes a first member 70 and a second member 71 that are rotatable relative to each other and a space portion 75 inside the torque device 60. The space portion 75 is continuous through the first member 70 and the second member 71. The guide wire 10 can be inserted through the space portion 75.

The torque device 60 is formed with an opening portion (not shown) continuous over the first member 70 and the second member 71. The opening portion connects the space portion 75 and the outside in the radial direction as in the first embodiment.

The cylindrical first member 70 has an outer peripheral surface 80 and an inner peripheral surface 81, and a part of the inner peripheral surface 81 is formed by an inner peripheral member 82. The inner peripheral member 82 is formed of an elastic material such as rubber, and an inner peripheral surface 82a constitutes a part of a peripheral surface of the space portion 75. One end portion of the inner peripheral member 82 in the length direction is a fixed portion 82b fixed to the first member 70, and the other end portion of the inner peripheral member 82 in the length direction is a pressure contact portion 82c that is in pressure contact with the second member 71. Further, on an inner peripheral side relative to the pressure contact portion 82c, the inner peripheral member 82 is provided with a hard portion 82d in pressure contact with an inner peripheral member 92 provided on the second member 71. The inner peripheral member 82 is made of an elastic and soft material, and the hard portion 82d is made of a hard material.

The second member 71 has an outer peripheral surface 90 and an inner peripheral surface 91, and a part of the inner peripheral surface 91 is formed by the inner peripheral member 92. The inner peripheral member 92 is formed of an elastic material such as rubber like the inner peripheral member 82 of the first member 70, and an inner peripheral surface 92a constitutes a part of the peripheral surface of the space portion 75. One end portion of the inner peripheral member 92 in the length direction is a fixed portion 92b fixed to the second member 71, and the other end portion of the inner peripheral member 92 in the length direction is a pressure contact portion 92c that is in pressure contact with the hard portion 82c provided on the inner peripheral member 82 of the first member 70.

The first member 70 and the second member 71 can rotate relative to each other via a rotation latch section 76. As shown in Fig. 6, the rotation latch section 76 includes a gear portion 76a formed on the first member 70 and a claw portion 76b provided on the second member 71. The claw portion 76b is attached to the second member 71 via an biasing portion 76c such as a spring, and the claw portion 76b is biased in a direction of being pressed against the gear portion 76a. The gear portion 76a includes saw blade-shaped uneven portions along a circumferential direction, and each uneven portion has a surface inclined along the circumferential direction and a surface substantially along the radial direction. The claw portion 76b has an acute-angled tip portion, and one side thereof is substantially along the radial direction. Therefore, when the first member 70 rotates with respect to the second member 71 in a direction of an arrow in Fig. 6, the claw portion 76b can ride on the inclined surface of the gear portion 76a. When the first member 70 tries to rotate with respect to the second member 71 in a direction opposite to the arrow in Fig. 6, the side of the claw portion 76b that is substantially along the radial direction is abutted against a surface substantially along the radial direction of the gear portion 76a, and therefore, the first member 70 cannot rotate. Accordingly, the first member 70 can rotate only in one direction with respect to the second member 71 and the rotation state can be maintained.

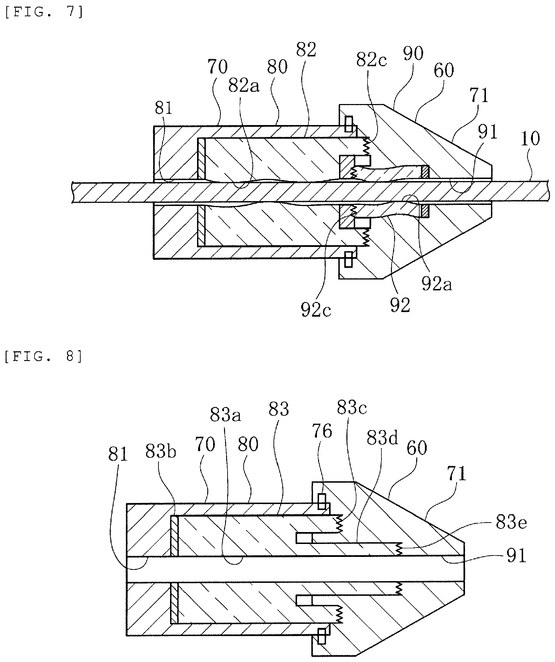

When the first member 70 and the second member 71 are rotated relative to each other, since both end portions of the inner peripheral member 82 of the first member 70 in the length direction are restrained correspondingly by the fixed portion 82b and the pressure contact portion 82c, twisting occurs along the length direction. Along with this, uneven portions are generated on the inner peripheral surface 82a of the inner peripheral member 82. Similarly, the inner peripheral member 92 of the second member 71 is twisted along the length direction, and uneven portions are generated on the inner peripheral surface 92a. Accordingly, as shown in Fig. 7, an inner diameter of the space portion 75 is decreased, and the guide wire 10 inserted into the space portion 75 can be held. That is, the inner peripheral members 82, 92 can function as diameter decreasing portions. Accordingly, the guide wire 10 is fixed in the length direction and the radial direction with respect to the torque device 60, and the operator can move the guide wire 10 forward and backward, and can rotate the guide wire 10 via the torque device 60.

Accordingly, the first member 70 and the second member 71 are provided with the inner peripheral members 82, 92 whose both end portions in the length direction are restrained, respectively, and the first member 70 and the second member 71 are rotated relative to each other. Therefore, the guide wire 10 can be easily held by the rotary operation by twisting the inner peripheral members 82, 92 to decrease the diameter of the inner peripheral surfaces 82a, 92a.

Further, a configuration of the inner peripheral member is not limited thereto. As shown in Fig. 8, an inner peripheral member 83 of the first member 70 has the pressure contact portion 82c that is in pressure contact with the second member 71 at an outer peripheral portion. Further, an inner peripheral surface 83a of the inner peripheral member 83 includes a protruding portion 83d that enters toward the second member 71. The protruding portion 83d enlarges the inner peripheral surface 83a of the inner peripheral member 83 to the inner peripheral surface 91 of the second member 71. A tip portion of the protruding portion 83d is a second pressure contact portion 83e that is in pressure contact with the second member 71. Accordingly, the inner peripheral member 83 provided on the first member 70 may have the inner peripheral surface 83a extending over the first member 70 and the second member 71. In Fig. 8, the inner peripheral member 83 is provided on the first member 70, and an inner peripheral member may be provided on the second member 71 such that an inner peripheral surface thereof enters the first member 70.

As described above, the torque device 20 according to the present embodiment that holds the elongated body 10 includes: the first member 30 and the second member 31 that define the continuous space portion 35 through which the elongated body 10 is inserted along the length direction. The first member 30 and the second member 31 are rotatable relative to each other while holding the elongated body 10, and have the opening portion 36 that connects the space portion 35 and the outside in the radial direction. The opening portion 36 is continuous in the length direction of the first member 30 and the second member 31, and has the width through which the elongated body 10 is insertable. At least one of the first member 30 and the second member 31 includes the diameter decreasing portion 45 that decreases the diameter of the space portion 35 by the first member 30 and the second member 31 rotating relative to each other. Accordingly, the torque device 20 according to the present embodiment can easily guide the elongated body 10 from the opening portion 36 to the space portion 35, and can hold the elongated body 10 disposed in the space portion 35 by the first member 30 and the second member 31 rotating relative to each other, and therefore, the elongated body 10 can be easily attached and held. Further, the elongated body 10 attached and held by the torque device 20 can be moved forward and backward and rotated, and the operability can be improved.

Further, the first member 30 and the second member 31 may move along the length direction of the elongated body 10 by rotating relative to each other, and the diameter decreasing portion 45 of one of the first member 30 and the second member 31 may be decreased in diameter by the other one of the first member 30 and the second member 31 moving along the length direction of the elongated body 10. Accordingly, it is possible to form a structure in which the diameter decreasing portion 45 is decreased with a simple structure of forming a screw surface on the first member 30 and the second member 31.

Further, the diameter decreasing portion 45 may be elastically deformable in the radial direction, and may have the pressing surface portion 48 that is pressed in the radial direction by the other one of the first member 30 and the second member 31 when the first member 30 and the second member 31 move along the length direction of the elongated body 10 by rotating relative to each other. Accordingly, the elongated body 10 can be elastically held by the diameter decreasing portion 45, and the operability of the held elongated body 10 can be further improved.

Further, at least one of the first member 70 and the second member 71 may have the elastically deformable inner peripheral member 82 that is in pressure contact with or joined to the other one of the first member 70 and the second member 71, and the inner peripheral member 82 may have the inner peripheral surface 82a that constitutes at least a part of the peripheral surface of the space portion 75 to form the diameter decreasing portion. Accordingly, by the first member 70 and the second member 71 rotating relative to each other, it is possible to form a structure in which the inner peripheral member 82 is twisted to decrease the diameter.

Further, the inner peripheral member 83 provided on one of the first member 70 and the second member 71 may have the protruding portion 83d that enters the other one of the first member 70 and the second member 71. Accordingly, the inner peripheral surface 83a of the inner peripheral member 83 can be enlarged and the elongated body 10 can be held more firmly.

Further, the opening portion 36 may be continuous in the length direction of the elongated body 10 over the first member 30 and the second member 31 in a state where the diameter decreasing portion 45 is enlarged. Accordingly, the elongated body 10 can be easily inserted into the space portion 35 through the opening portion 36.

Note that the invention is not limited to the embodiments described above and various modifications can be made by those skilled in the art within a scope of the technical idea of the invention. For example, in the above-described embodiment, the elongated body is the guide wire 10. Alternatively, the elongated body is not limited to the guide wire 10 as long as the elongated body is a member that is long in the length direction and is operated to move forward and backward and rotate and can withstand a pressure of the diameter decreasing portion 45.

Further, the first member 30 of the torque device 20 shown in Fig. 1 has a nut-shaped outer peripheral surface, and may have an outer peripheral surface having a shape other than the above shape as long as the first member 30 has a shape that is easy to operate.

Further, in the embodiments described above, the guide wire 10 is attached to the torque device 20 via the opening portion 36, and the guide wire 10 may be attached to the torque device 20 in advance. In this case, after moving the torque device 20 to any position in the length direction of the guide wire 10, the first member 30 and the second member 31 are rotated to hold the guide wire 10, and the guide wire 10 is operated via the torque device 20.

Further, in the torque device 60 of the second embodiment, the inner peripheral surfaces 82a, 92a of the inner peripheral members 82, 92 are smooth surfaces, and protruding portions and uneven portions may be provided to make it easier to hold the guide wire 10.

This application is based on Japanese Patent Application No. 2019-45466, filed on March 13, 2019, the disclosure of which is referenced and incorporated as a whole.

### Reference Signs List

- 10:: guide wire
- 20:: torque device
- 30:: first member
- 31:: second member
- 35:: space portion
- 36:: opening portion
- 40:: outer peripheral surface
- 41:: inner peripheral surface
- 42:: inner peripheral screw surface
- 45:: diameter decreasing portion
- 46:: outer peripheral surface
- 47:: inner peripheral surface
- 48:: pressing surface portion
- 50:: outer peripheral surface
- 51:: outer peripheral screw surface
- 52:: inner peripheral surface
- 54:: tapered surface portion
- 70:: first member
- 71:: second member
- 75:: space portion
- 76:: rotation latch section
- 80:: outer peripheral surface
- 81:: inner peripheral surface
- 82:: inner peripheral member
- 82a:: inner peripheral surface
- 82b:: fixed portion
- 82c:: pressure contact portion
- 82d:: hard portion
- 84:: slope surface portion
- 90:: outer peripheral surface
- 91:: inner peripheral surface
- 92:: inner peripheral member

## Claims

1. A torque device that holds an elongated body, the torque device comprising:
a first member and a second member that define a continuous space portion through which the elongated body is inserted along a length direction, wherein
the first member and the second member are rotatable relative to each other while holding the elongated body, and have an opening portion that connects the space portion and outside in a radial direction,
the opening portion is continuous in a length direction of the first member and the second member, and has a width through which the elongated body is insertable, and
at least one of the first member and the second member includes a diameter decreasing portion that decreases a diameter of the space portion by the first member and the second member rotating relative to each other.

2. The torque device according to claim 1, wherein
the first member and the second member move along the length direction of the elongated body by rotating relative to each other, and
the diameter decreasing portion of one of the first member and the second member is decreased in diameter by the other one of the first member and the second member moving along the length direction of the elongated body.

3. The torque device according to claim 2, wherein
the diameter decreasing portion is elastically deformable in the radial direction, and has a pressing surface portion that is pressed in the radial direction by the other one of the first member and the second member when the first member and the second member move along the length direction of the elongated body by rotating relative to each other.

4. The torque device according to claim 1, wherein
at least one of the first member and the second member has an elastically deformable inner peripheral member that is in pressure contact with or joined to the other one of the first member and the second member, and the inner peripheral member has an inner peripheral surface that constitutes at least a part of a peripheral surface of the space portion to form the diameter decreasing portion.

5. The torque device according to claim 4, wherein
the inner peripheral member provided on one of the first member and the second member includes a protruding portion that enters the other one of the first member and the second member.

6. The torque device according to any one of claims 1 to 5, wherein
the opening portion is continuous in the length direction of the elongated body over the first member and the second member in a state where the diameter decreasing portion is enlarged.
